# EUROPEAN PATENT APPLICATION

(11) **EP 1 510 195 A1**
(43) Date of publication of application: **02.03.2005**
(21) Application number: 03019740.4
(22) Date of filing: 29.08.2003
(51) Int. Cl.: A61H 39/00

(54) **Stimulating device for stimulating muscle and vital points of a human body**

(71) Applicant: Chen, Yi-Ying, Vancouver, British Columbia (CA)
(72) Inventor: Wang, Wei-Cheng, Panchiao city, Taipei Hsien (TW)
(74) Representative: Casalonga, Axel

(57) **Abstract**

A stimulating device for activating muscles and vital points of a human body includes a fixing element (10), electrical plates (11,12) received in the fixing element (10), a thermal device received in the fixing element (10), a control circuit electrically connected to the electrical plates (11,12) to generate low frequency signal to the electrical plates (11,12) such that when the electrical plates (11,12) are placed on top of the vital points, the low frequency signal to the electrical plates (11,12) is able to provide stimulus to the vital points. The control circuit is further electrically connected to a resistance (13) of the thermal device so as to stimulate and provide a thermal effect to surrounding tissues and muscles.

## Description

### 1. Field of the Invention

The present invention relates to an insole stimulating device that includes the electrical stimulating device, magnetic unit and thermal unit to perturb intercellular fluid, to excite the cellular permeability and to increase ion flow of stimulated muscle as well as the vital points of the human body. Our current invention is to apply large area electrodes to areas of body that also contain well defined vital points, so that, with a carefully controlled electronic stimulation, it can effect a remote treatment of body parts or functions through the combination of local muscular stimulations and peripheral nervous stimulation. Moreover, the stimulating device having a pair of electrical plate to provide electrical field that agitates ion flow, the magnetic unit to provide field that enhances intercellular ion flow and thermal unit to provide the thermal effect to the local area to improve status of circulation. Also, said the stimulating device that is able to enhance Chi and intercellular fluid circulation at the local area that will alleviate edema at lower limb and will speedup the healing process for low back pain.

The electrical plates fixed on the insole are placed against the skin of the human body and aligned with the vital points or area of pressure point, K1 and foot heel area (FHA, a newly found singular vital point) of the human body. A controlled electric stimulating circuit is connected to the electrical plates to provide oscillation pulses string or a continuous modulating AC signal to the electrical plates. So that the oscillation pulses string from the electrical plates is able to stimulate the local area of target muscle group as well as the vital points. This invention is much effective than an example of others treatments that are indicated in the treatment of the lower back pain. A local electronic massaging of the waist can be effective in the temporary relief of the lower back pain, but the pain relief is usually temporary and not sustainable and returns soon after the treatment ends.

While there are many causes for the lower back pain, a good portion of such a pain is the result of progressive deterioration of muscle and nerve systems from the feet up to the waist. The traditional practice of acupuncture sometimes also is accompanied by the application of heat to the acupuncture points. Such heat may enhance the effectiveness of the needle punctuation. No known electronic stimulation systems integrate the ability to apply heat to the point of stimulation, even though there are many stand alone heat applying systems, such as heating pads, for such symptoms of muscle pain, back pain, etc.

When we invent the insole stimulation system using large-area electrodes on two locations on the foot containing the vital K1 and FHA acupuncture points, the effect of the treatment has been demonstrated to greatly exceed that of each method administered separately. It is also commonly known that stimulation vital acupuncture points, K1 and FHA, on each foot, with needles or electronic stimulation, can provide great relief in the lower back pain. We use sophisticated electronic technologies to effect electronic, thermal and magnetic stimulations. It is further noticed that, the thermal stimulation is not applied directly to the lower back. Instead it is applied to the areas where electronic stimulation is applied to, the same area where vital acupuncture points lie. This is because electrical stimulation will dramatically enhance intercellular permeability and increase ion flow of stimulated muscle which improves lower body circulation that will relieve lower back pain.

The magnetic units are customarized in the fixing element on the insole that is corresponding to the electrical plates to facilitate the increase of intercellular ion flow at the local area of the human body.

A thermal element, such as a heating device, is customary in the fixing element on the insole that is connected to a thermal control unit to provide thermal effect to the local body area at skin to improve the status of circulation. A thermal control unit is connected to the power source that provides the temperature control to the heat element. So that the thermal effect from the heat unit warms the surrounding muscular tissues of the skin to facilitate the increase of the blood circulation of the local area.

### 2. Description of Related Art

Electronic Stimulation has been applied to muscle groups for temporary relief of pain, physical training or rehabilitation. It has also been applied to peripheral nerve system for temporary relief of pain. These applications have generally been applied to the body where actual pain was experienced. The areas of the electrodes used in these applications tend to be rather large in order to provide a broad range of stimulation to a group of muscle or nerves. Therefore, the prior art of electronic stimulation has consisted two distinctly different schools of applications: (1) electronic stimulation using large sized electrodes spaced from each other to effect a significant local muscular and nervous stimulation; and, (2) electronic stimulation using small electrodes spaced very close to each other to effect precise vital point nervous stimulation to effect treatment or pain relief of remote body parts or functions.

Acupuncture has been a traditional remedy for Chinese who may suffer aches and pains etc. The acupuncturist applied needles that extend into vital points of a patient so as to help the patient to enhance the 'Chi' and circulation of blood. The vital points are over the body and also known as the pressure points. Each of the vital points is the junction of Chi and blood. Therefore, every vital point dominates a primary task concerning the health of the human.

The primary purpose of acupuncture is to stimulate the vital points by needles such that the channels for 'Chi' are cleared. In spite of the acknowledged effect of acupuncture, the only problem is that an appointment needs to be made first with the acupuncturist. That is, the patient's day somehow has to be rescheduled to match the acupuncturist's availability. To overcome the shortcoming, an easy way of achieving the similar effect is to use the magnetic units. The magnetic units, energy stone, are placed on top of some specific vital points. The energy stone constitutes an energy field around the vital point so as to facilitate the blood circulation, which effectively relieves the patient's suffering.

Recent electronic stimulation application also include applying the stimulation to particular" acupuncture" points ( or vital points ) to simulate the effect of needles used in the acupuncture for the relief of pain or treatment of a wide variety of diseases. In such an application, the points of stimulation may be different from the target of the treatment. An example of such an appl ication is in US patent 4981146, entitled " Nausca control Device", where a closed spaced electrodes carrying pre-determined level of electronic stimulation is applied to the vital point per-cardium six acupuncture point, generally called P6 point in acupuncture, under the wrist, to effect the suppression of motion sickness, morning sickness, vomiting, etc.

Another Chinese remedy is massage. Using appropriate force that applies to muscles or some specific vital points is able to relieve the patient's pain, fatigue and many other disorders. However, since massaging is very tiring for the masseur, an electronic massager is developed to the market. With the electronic massager, the user is able to relax whenever is suitable.

However, to have both effects from the electronic massager and the energy stone seems impossible unless there is a way to integrate the space, signal and the circuitry of the electronic massager and the energy stone.

To overcome the shortcomings, the present invention tends to provide an improved stimulating device to mitigate and obviate the aforementioned problems.

The primary objective of the present invention is to provide an improved stimulating device to enhance the intercellular ions circulation. The present invented device includes the electrical plates provide stimulus to the vital points as well as the muscles. A heating element provides thermal effect to the area, where the heat element is applied to improve the status of local circulation. A magnetic unit aligned with the electrical plates to that the magnetic field from the magnetic units is able to facilitate the increase of intercellular ion circulation of a human to whom is wearing the stimulating device of the present invention.

Another objective of the present invention is to provide an improved control circuit to not only provide electricity to the electrical plates, but also regulate the effect of the resistance of the stimulating device of the present invention so that the patient is able to adjust the control circuit according to individual physical conditions.

Other objectives, advantages and novel features of the invention will become more apparent from the following detailed description when taken in conjunction with the accompanying drawings.

In the drawings
Fig. 1 is a perspective view showing an insole, a preferred embodiment of the stimulating device being received in a shoe;
Fig. 2 is an exploded perspective view of the stimulating device and an embodiment of the fixing element in the shoe in Fig. 1;
Fig. 3 is a schematic view in partial section showing the relative positions between the stimulating device and the fixing element;
Fig. 4 is a block diagram of the control circuit of the present invention;
Figs. 5A and 5B are schematic views showing the output wave patterns after being converted from low frequency signal;
Fig. 6 is a schematic view showing the application of the stimulating device of the present invention; and
Figs. 7, 8 and 9 are schematic views showing the stimulating device is applied to different parts of a patient.

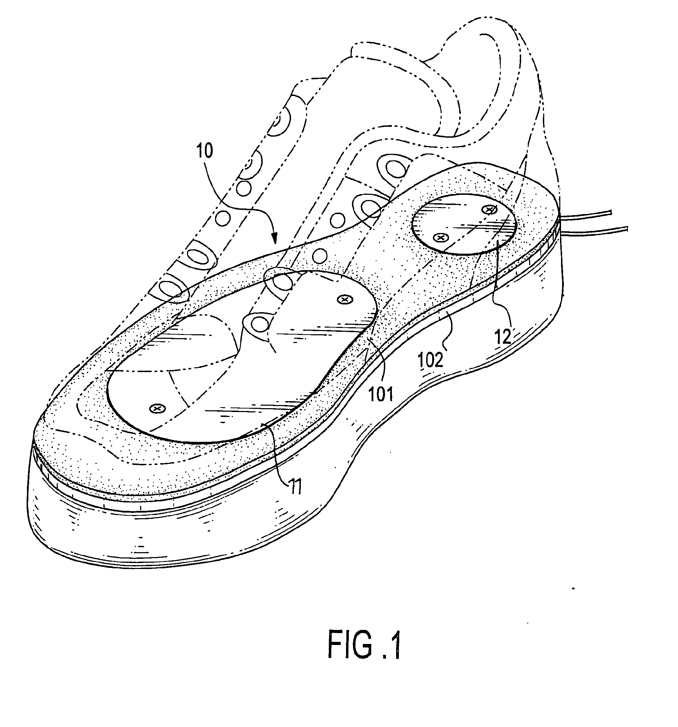

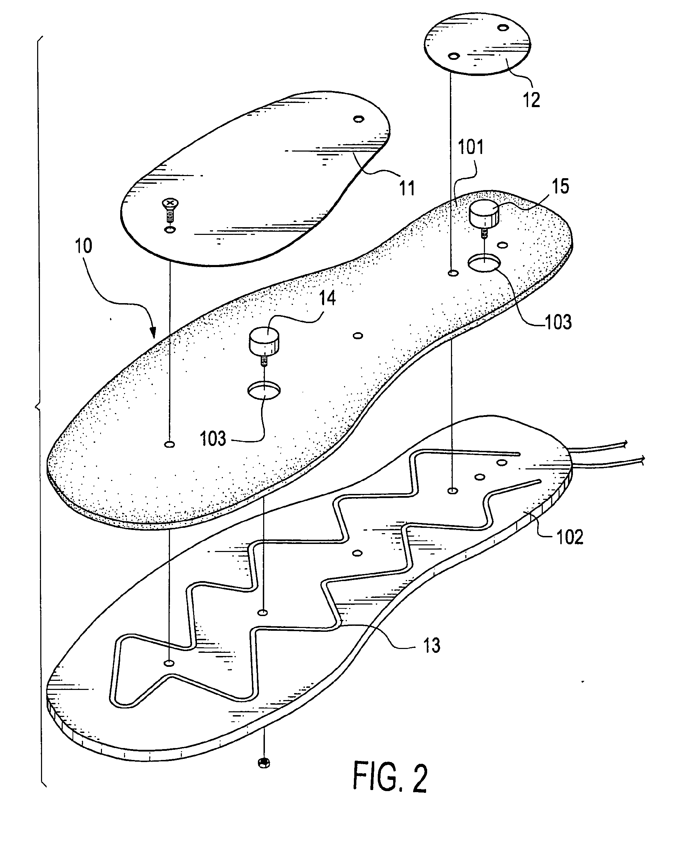

With reference to Figs. 1 and 2, the stimulating device in accordance with the present invention includes a fixing element (10), at least one pair of electrical plates (11,12), a heat element (13), at least one magnetic stone (14,15) and a control circuit (not shown).

With reference to Fig. 2 again that the fixing element (10) in this embodiment includes a pad (101) and an insole (102) securely connected to the pad (10). The pad (10) has holes (103) corresponding to the magnetic units (14,15) such that the magnetic units (14,15) are able to be received in the holes (103). The resistance (13) is substantially located along a contour of the insole (102). The electrical plates (11,12) are respectively then securely applied on top of the magnetic units (14,15). Bolts (not numbered) are used to firmly engage the pad (10) to the insole (102). It is to be noted that the electrical plates (11,12) may have one positive plate and one negative plate. Other embodiments show that the combination of the electrical plates (11,12) may have one positive plate and the others arc negative plates or vice versa.

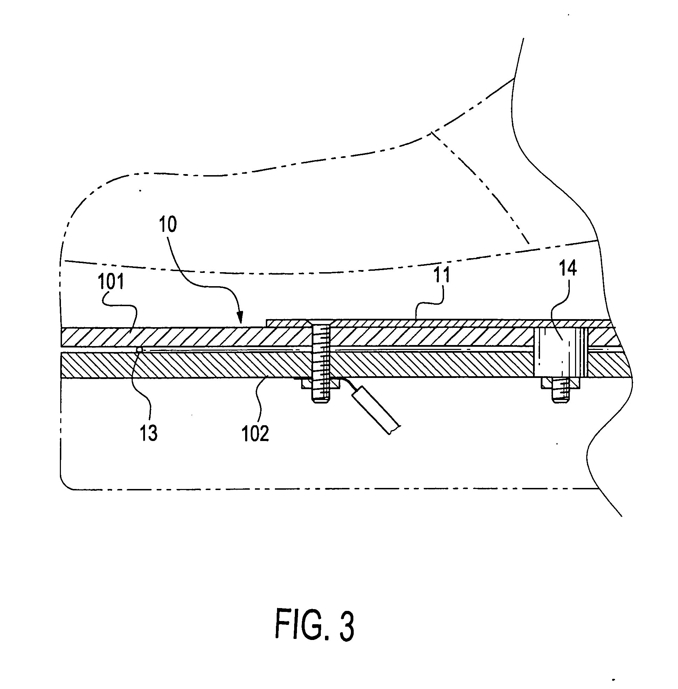

With reference to Fig. 3, after the assembly of the stimulating device of the present invention, the electrical plates (11,12) (only the electrical plate (11) is shown) correspond to the magnetic units (14,15) (only the energy stone (14) is shown).

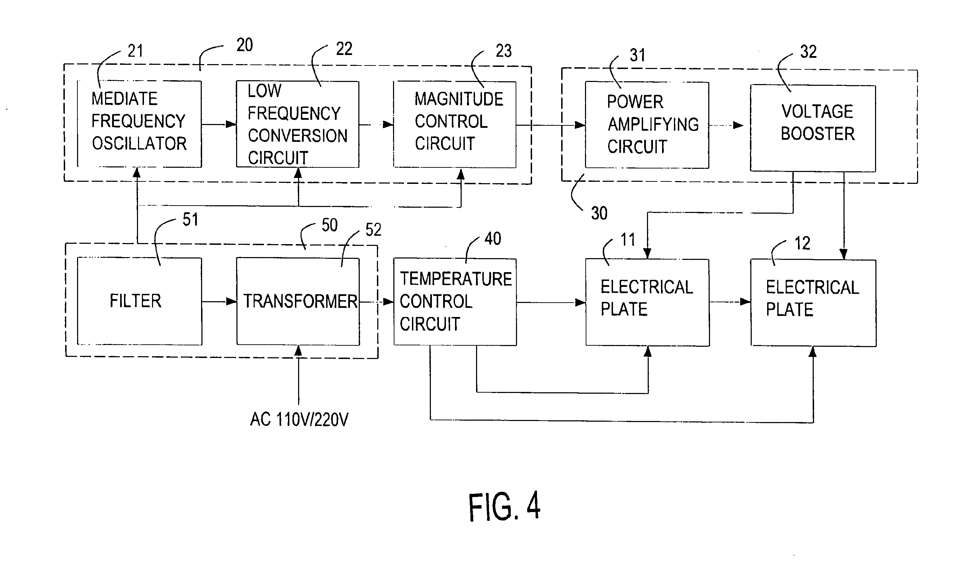

With reference to Fig. 4, the control circuit of the present invention includes a signal generating unit (20), a power amplifying/boosting circuit (30), a temperature control circuit (40) and a power source (50).

The signal generating unit (20) generates a mediate or a low frequency signal and sends the generated signal to the electrical plates (11,12) so as to stimulate the muscle and vital points around the electrical plates (11,12). The signal generating unit (20) includes a mediate frequency oscillator (21), a low frequency conversion circuit (22) and a magnitude control circuit (23). The mediate frequency oscillator (21) is to generate a mediate frequency signal which is then converted to a low frequency signal by the low frequency conversion circuit (22). The magnitude control circuit (23) aims to control the strength of the converted low frequency signal.

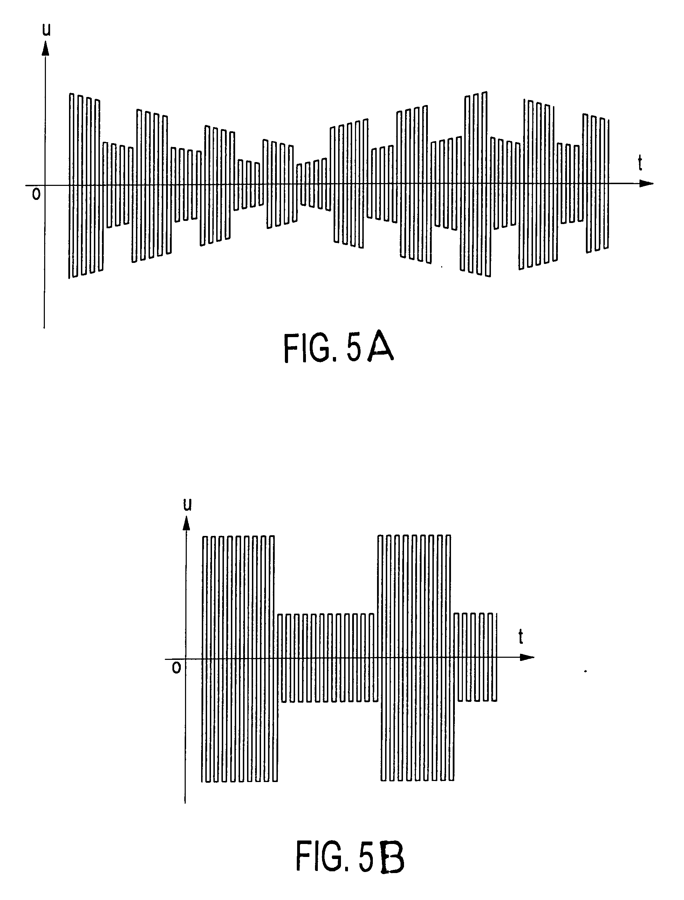

With reference to Figs. 5A and 5B, the drawings show two different kinds of working waves for the magnitude control circuit (23) by the low frequency conversion unit (22).

The power amplifying/boosting circuit (30) connecting to the output of the signal generating unit (20) includes a power amplifying circuit (31) and a voltage booster (32). The signal sent by the signal generating unit (20) is processed respectively by the power amplifying circuit (31) and the voltage booster (32) and sent to the electrical plates (11,12).

The temperature control circuit (40) includes an output connecting to the resistance (13) for controlling the temperature of the fixing element (10).

The power source (50) is composed of a filter (51) and a transformer (52). The transformer (52) provides electricity to the resistance (13) via the temperature control circuit (40) and rectifies the current from the filter (51).

Therefore, from the foregoing description, the preferred embodiment of the present invention shows that the fixing element (10) is composed of a pad (shoe pad) and an insole (102) such that the user is able to place the fixing element (10) inside the shoe with the heat element (13) substantially surrounding the contour of the insole (102) and the electrical plates (11,12) and the magnetic units (14,15) received in the pad (101). With the foregoing arrangement, the user is able to simultaneously stimulate and provide mild thermal effect to the vital points.

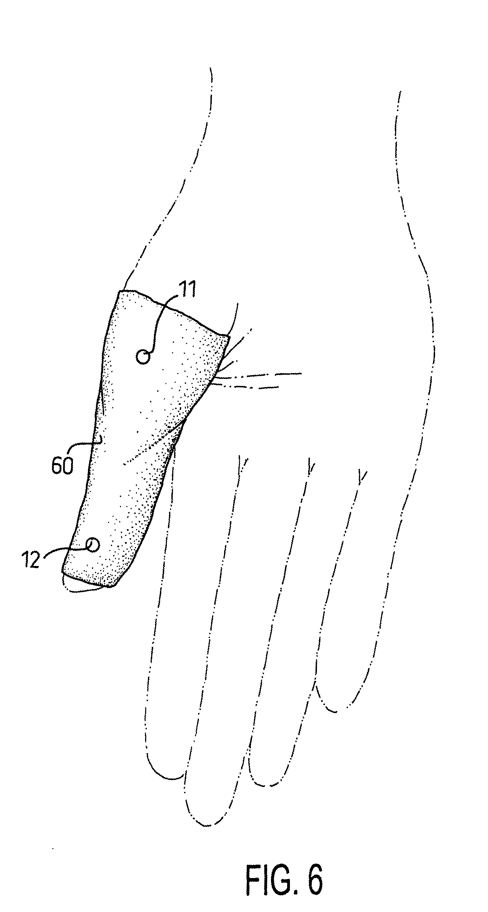

With reference to Fig. 6, the fixing element (60) may be formed by elastic cloth to be worn on a finger. The positive electrical plate (11) and the negative electrical plate (12) respectively have a corresponding magnetic stone (14,15) which corresponds to various vital points on the finger. Thus, the user is able to stimulate the muscle and stimulate the surrounding tissues surrounding the specific vital points by the electrical plates (11,12) and the energy stones (14,15).

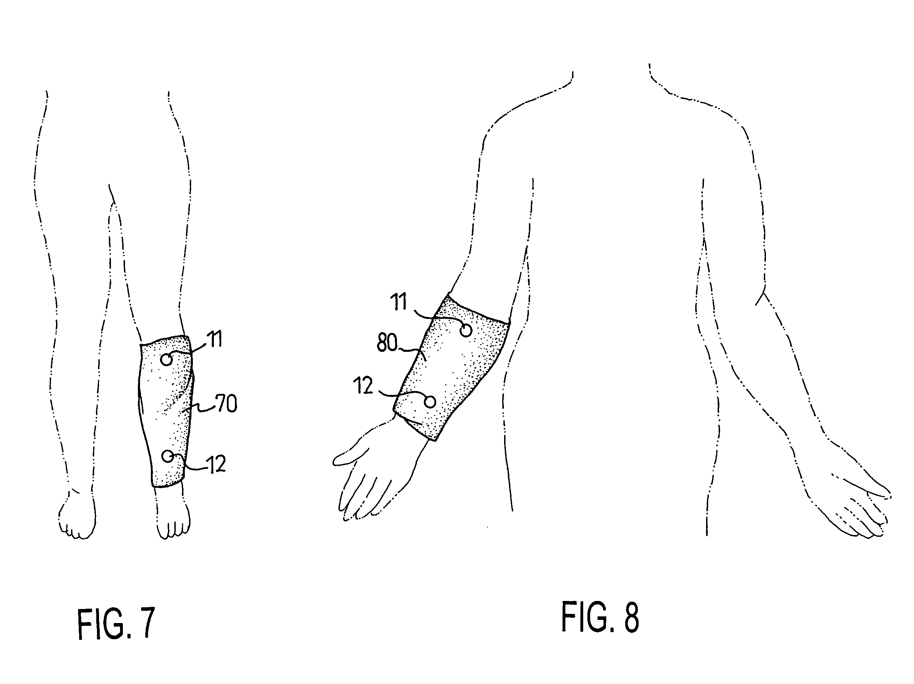

With reference to Figs. 7 and 8, the fixing element (70,80) also may be made of an elastic cloth so that the user is able to wear the fixing element (70,80) on the leg or on the arm to respectively stimulate and warm the specific vital points.

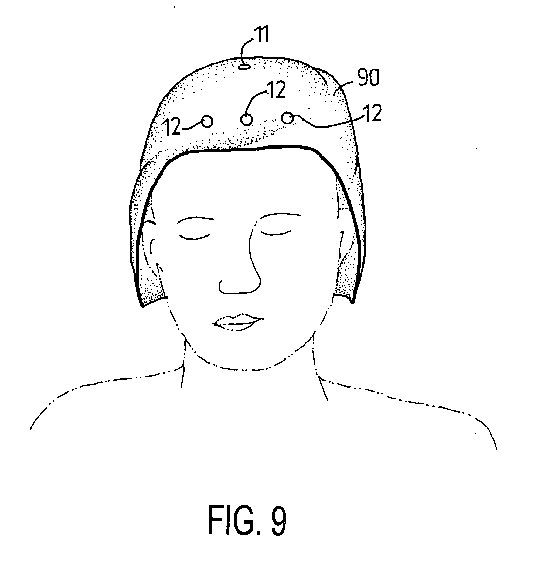

With reference to Fig. 9, the fixing element (90) may be a cap. Because the vital points in the head are more complex than other portions of the body, the arrangement of the electrical plates (11,12) may be one positive plate and one negative plate, one positive plate and others negative plates or one negative plate and others positive plates.

Therefore, with the temperature provided by the resistance (13), the specific portion of the patient's body is treated for swelling and the like. Also the electrical plates (11,12) provide a massaging effect to the vital points and surrounding muscles with the assistance of the power amplifying circuit (31) and the voltage booster (32). The effect by the resistance (13) is much the same as the "warm-up" before starting a sport, which is able to increase the blood circulation, the flexibility of the body and metabolism of the body.

It is to be understood, however, that even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. An insole stimulating device for activating muscular contraction, stimulating vital points of a human body and enhance local intercellular ion circulation, the stimulating device comprising:
a fixing element (10);
electrical plates (11,12) received in the fixing element (10);
a thermal device received in the fixing element (10); and
a control circuit electrically connected to the electrical plates (11,12) and the thermal device to generate low frequency signal to the electrical plates (11,12) such that when the electrical plates are placed on top of the vital points, the low frequency signal to the electrical plates is able to provide stimulus to the vital points and to stimulate and provide a thermal effect to surrounding tissues and muscles.

2. The stimulating device as claimed in claim 1, wherein the fixing element (10) is composed of an insole (102) and a pad (101) securely connected to the insole (102), the resistance (13) is received in the insole (102) and the electrical plates (11,12) are received in the pad (101).

3. The stimulating device as claimed in claim 1 further comprising magnetic units (14,15) each corresponding to one of the electrical plates.

4. The stimulating device as claimed in claim 2 further comprising magnetic units (14,15) each corresponding to one of the electrical plates (11,12).

5. The stimulating device as claimed in claim 1, wherein the fixing element (10) is made of an elastic cloth adapted to be worn on a portion of the human body.

6. The stimulating device as claimed in claim 2, wherein the fixing element (10) is made of an elastic cloth adapted to be worn on a portion of the human body.

7. The stimulating device as claimed in claim 4, wherein the fixing element (10) is made of an elastic cloth adapted to be worn on a portion of the human body.

8. The stimulating device as claimed in claim 1, wherein the control circuit comprises:
a signal generating unit (20) composed of a mediate frequency oscillator (21), a low frequency conversion circuit (22) and a magnitude control circuit (23), wherein the mediate frequency oscillator (21) generates a mediate frequency signal which is then converted to a low frequency signal by the low frequency conversion circuit (22), the magnitude control circuit (23) aims to control the strength of the converted low frequency signal to the electrical plates (11,12),
a power amplifying/boosting circuit (30), connecting to the output of the signal generating unit (20), including a power amplifying circuit (31) and a voltage booster (32), wherein the signal sent by the signal generating unit (20) is processed respectively by the power amplifying circuit (30) and the voltage booster (32) and sent to the electrical plates (11,12),
a temperature control circuit (40) including an output connecting to the resistance (13) for controlling the temperature of the fixing element (10); and
a power source (50) composed of a filter (51) and a transformer (52), wherein the transformer (52) provides electricity to the resistance (13) via the temperature control circuit (40) and rectifies the current from the filter (51).

9. The stimulating device as claimed in claim 4, wherein the control circuit comprises:
a signal generating unit (20) composed of a mediate frequency oscillator (21), a low frequency conversion circuit (22) and a magnitude control circuit (23), wherein the mediate frequency oscillator (21) generates a mediate frequency signal which is then converted to a low frequency signal by the low frequency conversion circuit (22), the magnitude control circuit (23) aims to control the strength of the converted low frequency signal to the electrical plates (11,12),
a power amplifying/boosting circuit (30), connecting to the output of the signal generating unit (20), including a power amplifying circuit (31) and a voltage booster (32), wherein the signal sent by the signal generating unit (20) is processed respectively by the power amplifying circuit (30) and the voltage booster (32) and sent to the electrical plates (11,12),
a temperature control circuit (40) including an output connecting to the resistance (13) for controlling the temperature of the fixing element (10); and
a power source (50) composed of a filter (51) and a transformer (52), wherein the transformer (52) provides electricity to the resistance (13) via the temperature control circuit (40) and rectifies the current from the filter (51).

10. The stimulating device as claimed in claim 1, wherein the electrical plates (11,12) comprise one positive plate and one negative plate.

11. The stimulating device as claimed in claim 1, wherein the electrical plates (11,12) comprise one positive plate and negative plates.

12. The stimulating device as claimed in claim 1, wherein the electrical plates (11,12) comprise one negative plate and positive plates.

13. The stimulating device as claimed in claim 8, wherein the electrical plates (11,12) comprise one positive plate and one negative plate.

14. The stimulating device as claimed in claim 9, wherein the electrical plates (11,12) comprise one positive plate and one negative plate.
